Europäisches Patentamt

European Patent Office

Office européen des brevets

(19)

(11) EP 0 736 324 B1

(12) EUROPÄISCHE PATENTSCHRIFT

(45) Veröffentlichungstag und Bekanntmachung des Hinweises auf die Patenterteilung:
26.09.2001 Patentblatt 2001/39

(51) Int Cl.⁷: **B01J 23/56**, C07C 68/00, C07C 69/96

(21) Anmeldenummer: 96104708.1

(22) Anmeldetag: 25.03.1996

(54) **Verwendung von Platinmetall enthaltenden Träger-Katalysatoren zur Herstellung von Diarylcarbonaten**

Use of supported platinum catalysts for the preparation of diaryle carbonates

Utilisation d'un catalyseur de platine supporté pour la préparation de carbonates de diaryle

(84) Benannte Vertragsstaaten:
DE ES FR GB IT NL

(30) Priorität: 05.04.1995 DE 19512618

(43) Veröffentlichungstag der Anmeldung:
09.10.1996 Patentblatt 1996/41

(73) Patentinhaber: BAYER AG
51368 Leverkusen (DE)

(72) Erfinder:
• Buysch, Hans-Josef, Dr.
47809 Krefeld (DE)
• Hesse, Carsten, Dr.
47800 Krefeld (DE)
• Jentsch, Jörg-Dietrich, Dr.
45468 Mülheim (DE)
• Rechner, Johann, Dr.
47906 Kempen (DE)
• Zirngiebl, Eberhard, Dr.
51061 Köln (DE)

(56) Entgegenhaltungen:
EP-A- 0 421 169        EP-A- 0 501 265
EP-A- 0 523 508        EP-A- 0 538 676
EP-A- 0 654 461

**Beschreibung**

Die vorliegende Erfindung betrifft Platinmetall enthaltende Träger-Katalysatoren und ihren Einsatz in Verfahren zur Herstellung von Diarylcarbonaten durch Umsetzung von aromatischen Hydroxyverbindungen mit Kohlenmonoxid und Sauerstoff, die dadurch gekennzeichnet sind, daß man pulverförmige oder geformte Träger verwendet, die unter Reaktionsbedingungen als Redoxkatalysatoren wirken können.

Es ist bekannt, organische Carbonate durch oxidative Umsetzung von aromatischen Hydroxyverbindungen mit Kohlenmonoxid in Gegenwart eines Edelmetall-Katalysators herzustellen (DE-OS 28 15 512). Als Edelmetall wird bevorzugt Palladium eingesetzt. Zusätzlich können ein Cokatalysator (z.B. Mangan- oder Kobaltsalze), eine Base, ein quartäres Salz, verschiedene Chinone bzw. Hydrochinone und Trockenmittel eingesetzt werden. Dabei kann in einem Lösungsmittel, bevorzugt Methylenchlorid, gearbeitet werden.

Für die wirtschaftliche Durchführung des im nächstliegenden Stand der Technik DE-OS 28 15 512 beschriebenen Prozesses ist neben der Aktivität und der Selektivität die effektive Wiedergewinnung des Edelmetall-Katalysators von entscheidender Bedeutung: Zum einen stellt der Edelmetall-Katalysator einen erheblichen Kostenfaktor dar. Verluste an Edelmetall-Katalysator müssen kostenintensiv ersetzt werden. Zum anderen dürfen keine Reste des Edelmetall-Katalysators im Produkt verbleiben. Für den Prozeß der oxidativen Carbonylierung von aromatischen Hydroxyverbindungen zu Diarylcarbonaten ist die wirtschaftliche und effiziente Rückgewinnung homogener Katalysatoren bisher nicht beschrieben. Mit geringerem Aufwand kann die Abtrennung eines Edelmetall-Katalysators aus einer flüssigen Reaktionsmischung z.B durch Filtrieren oder Zentrifugieren erfolgen, wenn man heterogene, z. B. Träger-Katalysatoren einsetzt.

Zur Herstellung von Träger-Katalysatoren allgemein geeignete Materialien sind bekannt. Je nach Art des Prozesses benutzt man Träger mit großer innerer Oberfläche, wie z.B. Aluminiumoxid, Magnesiumoxid, Aktivkohle oder Siliciumdioxid mit mehr als 50 $m^2$ Oberfläche pro Gramm oder Träger mit Oberflächen um 5 $m^2$/g und entsprechend größeren Porenradien, wie z.B. Ruß, Titandioxid, Eisenoxid oder Zinkoxid oder grobkörnige Träger, wie z.B. Siliciumcarbid und Korund (Ullmanns Enzyklopädie der technischen Chemie, 3.Auflage, Berlin/München 1957, Band 9, S.263 ff.). Grundsätzlich können sowohl synthetische Materialien, wie aktivierte Aluminiumoxide, Kieselgele, Silikate, Titandioxide oder Aktivkohlen als auch solche aus natürlichen Quellen, wie z.B. Bimsstein, Kaolin, Bleicherden, Bauxite, Bentonite, Kieselgur, Asbest oder Zeolithe verwendet werden.

In EP 572 980, EP 503 581 und EP 614 876 werden Edelmetall-Träger-Katalysatoren verwendet, die 5% Palladium auf Kohleträgern enthalten. Jedoch liefern derartige Träger-Katalysatoren nach eigenen Untersuchungen nur sehr unbefriedigende oder gar keine Umsätze, so daß auch diese für eine wirtschaftliche Prozeßführung nicht geeignet sind.

In EP-A 607 943 werden Cu-haltige Katalysatoren und deren Verwendung zur Herstellung von Kohlensäurediestern aus Alkoholen durch oxidative Umsetzung mit $O_2$ und CO beschrieben.

Auch diese Katalysatoren liefern jedoch nur sehr unbefriedigende Ausbeuten.

In JP 01/165 551 (zitiert nach C.A. 112 (1990), 76618j) wird erwähnt, daß für die Herstellung von aromatischen Carbonaten Palladium oder Pd-Verbindungen, wie Pd-acetylacetonat, in Kombination mit (Erd)Alkalijodiden oder Oniumjodiden, wie Tetrabutylammoniumjodid, und mindestens einem Zeolith eingesetzt werden können.

In JP 04/257 546 und JP 04/261 142 wird in je einem Beispiel ein Träger-Katalysator zur Herstellung von aromatischen Carbonaten beschrieben, bei dem SiliciumcarbidGranulat als Trägermaterial für einen Träger-Katalysator in einer Destillationskolonne verwendet wird. Obwohl in den betreffenden Beispielen unter drastischen Bedingungen (hoher Druck, hohe Temperatur) gearbeitet wird, ermöglicht dieser Katalysator nur sehr geringe Raum-Zeit-Ausbeuten. Diese niedrigen Raum-Zeit-Ausbeuten machen eine ökonomische Herstellung von aromatischen Carbonaten mit derartigen Träger-Katalysatoren unmöglich.

Es steht also bisher kein Träger-Katalysator zur Verfügung, mit dem Diarylcarbonate durch Umsetzung einer aromatischen Hydroxyverbindung mit Kohlenmonoxid und Sauerstoff ökonomisch effizient hergestellt werden können.

Es bestand daher die Aufgabe, einen Träger-Katalysator mit hoher Aktivität und Selektivität zu finden, der die ökonomisch effiziente Herstellung von Diarylcarbonaten durch Umsetzung einer aromatischen Hydroxyverbindung mit Kohlenmonoxid und Sauerstoff erlaubt.

Es wurde nun gefunden, daß die dargestellten Nachteile überwunden werden können, wenn Platinmetall-Trägerkatalysatoren verwendet werden, deren Träger Übergangsmetalloxide, wie zum Beispiel solche von V, Mn, Ti, Cu, La, der Seltenerdmetalle und deren Mischungen sind. Diese Übergangsmetalloxide werden erfindungsgemäß als Pulver, Tabletten oder bindemittelhaltige Extrudate eingesetzt. Geeignete Bindemittel sind z. B. $SiO_2$, $Al_2O_3$ oder Tonmineralien. Die Bindemittelgehalte können in einem breiten Bereich, beispielsweise von 0,5 bis 99,5 Gew. %, bezogen auf das Gesamtgewicht des Trägers, variiert werden. Die erfindungsgemäßen Träger sind nach bisheriger Vorstellung im Zusammenwirken mit der Platinmetall-Verbindung wie ein separat zugesetzter Cokatalysator wirksam; es werden jedoch alle Nachteile separat zugesetzter Cokatalysatoren vermieden, wie etwa die Vermischung mit dem Reaktionsprodukt und damit dessen Verunreinigung. Entsprechend der genannten Denkvorstellung handelt es sich bei allen genannten Metallen um solche, die in mehreren Wertigkeitsstufen auftreten können.

Die erfindungsgemäß verwendeten Träger-Katalysatoren enthalten im reaktionsbereiten Zustand ein Platinmetall, eine Platinmetall-Verbindung oder einen eine Platinmetall-Verbindung enthaltenden Komplex in einer Menge von 0,01 bis 15 Gew.-%, bevorzugt 0,05 bis 10 Gew.-%, gerechnet als Platinmetall und bezogen auf das Gesamtgewicht des Katalysators, auf einem Träger aus einem oder mehreren Oxiden der Metalle Ti, V, Mn, Cr, Fe, Co, Ni, Cu, La, Nb, Mo, Pb, der Seltenerd-metalle der Atomnummern 58 bis 71 und der Actiniden der Atomnummeren 89-92, bevorzugt aus einem oder mehreren Oxiden der Metalle Ti, V, Mn, Cr, Fe, Co, Ni Cu, La, Nb, Mo, Pb, Ce, Pr, Nd, Sm, Eu, Gd, Tb, Dy, Ho, Er, Tm, Yb, Lu und U.

Die Erfindung betrifft demnach die Verwendung des zuvor genannten Katalysators bei der zur Herstellung eines organischen Carbonats der Formel

$$R-O-CO-O-R \qquad (I),$$

in der

R   substituiertes oder nicht substituiertes $C_6$-$C_{12}$-Aryl, bevorzugt substituiertes oder nicht substituiertes Phenyl, besonders bevorzugt nicht substituiertes Phenyl bedeutet,

durch Umsetzung einer aromatischen Hydroxyverbindung der Formel

$$R-O-H \qquad (II),$$

worin R die oben angegebene Bedeutung hat,
mit Kohlenmonoxid und Sauerstoff in Gegenwart eines Cokatalysators, eines quartären Ammonium- oder Phosphonium-Salzes und einer Base bei 30 bis 200°C, bevorzugt 30 bis 150°C, besonders bevorzugt 40 bis 120°C und bei einem Druck von 1 bis 150 bar, bevorzugt 2 bis 50 bar, besonders bevorzugt 5 bis 25 bar.

Am Beispiel der Bildung von Diphenylcarbonat kann die dem erfindungsgemäßen Verfahren zugrundeliegende Reaktion wie folgt formelmäßig dargestellt werden:

$$2 \ C_6H_5\text{-}OH + CO + \tfrac{1}{2} \ O_2 \rightarrow (C_6H_5O)_2CO + H_2O$$

Für die erfindungsgemäße Verwendung als Katalysatorträger eignen sich u.a. Oxide folgender Metalle: Ti, V, Mn, Cr, Fe, Co, Ni, Cu, La, Nb, Mo, Pb, Seltenerd-Metalle der Atomnummern 58-71 und der Actiniden mit den Atomnummern 89-92 sowohl im Sinne chemisch einheitlicher Reinsubstanzen als auch im Gemisch. Insbesondere Oxide von La und den Seltenerdmetallen sind vielfach als Gemisch erhältlich, wie sie in der Natur vergesellschaftet vorkommen, und können so eingesetzt werden. Die den Oxiden zugrundeliegenden Metalle können in Form ihrer Kationen mindestens 2 verschiedene Wertigkeiten annehmen. In bevorzugter Weise sind dies ein oder mehrere Metalloxide aus der Gruppe von Ti, V, Mn, Cr, Fe, Co, Ni, Cu, La, Nb, Mo, Pb, Ce, Pr, Nd, Sm, Eu, Gd, Tb, Dy, Ho, Er, Tm, Yb, Lu und U.

Als besonders geeignet haben sich die Oxide des V, Mn, Ti, Co, Cu, La, der Seltenerdmetalle (Atomnummern 58-71) und ihre Mischungen herausgestellt.

Erfindungsgemäß verwendete Katalysatorträger können durch Fällen des gewünschten Metalloxids/Hydroxids, Waschen des Niederschlags zur Entfernung anorganischer Nebenprodukte und Trocknung hergestellt werden.

Wahlweise können getrocknete Katalysatorträger weiter durch Extrudieren, Tablettieren, gegebenenfalls unter Zumischen weiterer Katalysatorträger oder Bindemittel wie $SiO_2$ oder $Al_2O_3$, und Calcinieren modifiziert werden. Darstellung und Weiterverarbeitung der erfindungsgemäß verwendeten Katalysatorträger sind dem Fachmann wohl bekannt und Stand der Technik. Durch Anwendung der oben beschriebenen Präparationsmethoden erhält man im Reaktionsmedium unlösliche, poröse Feststoffe. Ihre Zusammensetzung variiert aufgrund der vielfältigen Abhängigkeiten von den Herstellbedingungen, wie Temperatur, Konzentration und Natur der Reaktanden, Geschwindigkeit und Reihenfolge der Reaktanden-Einbringung, pH Wert während der Präparation, Dauer der Fällung, Volumen und pH-Wert der Waschlösungen, Dauer und Temperatur von Trocknung und Calcinierung usw. Diese wechselnde Zusammensetzung der Metalloxide beeinflußt ihre Eignung als Katalysatorträger jedoch nur wenig. $TiO_2$-Träger können beispielsweise in der Modifikation des Rutils, Anatas oder Brookits, bevorzugt der des Rutils mit inneren Oberflächen von 2 bis 300, bevorzugt 5 bis 200 $m^2$/g eingesetzt werden.

Der Träger kann als Pulver oder Formkörper eingesetzt werden. Für den Fall der Anordnung des Träger-Katalysators als Festbett wird der Träger vorzugsweise als Formkörper, z.B. als Kugeln, Zylinder, Stäbchen, Hohlzylinder, Ringe, usw. eingesetzt.

Die Reaktivkomponente des Katalysators für die erfindungsgemäße Verwendung wird auf die genannten Metalloxide als Träger aufgebracht. Diese Reaktivkomponente besteht im reaktionsbereiten Zustand aus einem Platinmetall, einer Platinmetall-Verbindung oder einem eine Platinmetall-Verbindung enthaltenden Komplex, bevorzugt einem Platinmetall, einem Platinmetall-Halogenid oder einem ein Platinmetall-Halogenid enthaltenden Komplex, der außerdem beispielsweise Olefine, Amine, Phosphine, Nitrile, Kohlenmonoxid oder Wasser enthalten kann, wie A2(PdHal4), wobei A beispielsweise für Li, Na, K, $NH_4$, Rb, Cs, $NR_4$, worin R

für $C_6$- bis $C_{10}$-Aryl-, $C_7$- bis $C_{12}$-Aralkyl- und/oder $C_1$- bis $C_{20}$-Alkyl-Rest und Hal für ein Halogen, wie beispielsweise F, Cl, Br, I steht. Das Platinmetall liegt in einer Wertigkeitsstufe 0 bis 4 vor.

Solche Komplexverbindungen sind grundsätzlich bekannt. Beispiele sind: $Li_2(PdCl_4)$, $Na_2(PdCl_4)$, $K_2(PdCl_4)$, $(NBu_4)_2(PdCl_4)$, $Na_2(PdBr_4)$, $K_2(PdBr_4)$, $(NBu_4)_2(PdBr_4)$ mit (Bu = n-Butyl), Beispiele für olefinhaltige Platinmetallkomplexe sind [Allylpalladiumchlorid]-Dimer - $[C_3H_5PdCl]_2$, 1,5-Cyclooctadienpalladiumdichlorid - $C_8H_5PdCl_2$, Beispiele für phosphinhaltige Platinmetallkomplexe sind [1,2-Bis(diphenylphosphino)ethan]palladiumdichlorid $Pd[(C_6H_5)_2PCH_2CH_2P(C_6H_5)_2]Cl_2$, Bis(triphenylphosphino)palladiumdichlorid - $Pd[P(C_6H_5)_3]_2Cl_2$, Beispiele für aminhaltige Platinmetallkomplexe sind Diaminopalladiumdibromid -$Pd(NH_3)_2Br_2$, Diaminopalladiumdichlorid -$Pd(NH_3)_2Cl_2$, Tetraaminopalladiumtetrachloropalladat - $[Pd(NH_3)_4][PdCl_4]$, Beispiele für nitrilhaltige Platinmetallkomplexe sind Bis(acetonitril)palladiumdichlorid - $Pd(CH_3CN)_2Cl_2$, Bis(benzonitril)palladiumdichlorid, $Pd(C_6H_5CN)_2Cl_2$, Beispiele für kohlenmonoxidhaltige Platinmetallkomplexe sind Tetrabutylammoniumtribromocarbonylpalladat -$(NBu_4)Pd(CO)Br_3$ (mit Bu=n-Butyl) und Tetrabutylammoniumtrichlorocarbonylpalladat - $(NBu_4)Pd(CO)Cl_3$ (mit Bu = n-Butyl).

In den genannten Beispielen wurde Pd als Platinmetall genannt, jedoch kommen auch andere Platinmetalle in Betracht, wie Pt, Ir, Ru oder Rh; Pd und Rh sind jedoch bevorzugt, insbesondere Pd und liegen als Metall, Metallhalogenid oder Metallhalogenid enthaltende Komplexverbindung vor.

Es hat sich weiterhin gezeigt, daß das Platinmetall-Halogenid bzw. die das Platinmetall-Halogenid enthaltende Komplexverbindung in situ während der Präparation aus einer geeigneten halogenfreien Platinmetall-Verbindung und einer Halogenid enthaltenden Verbindung auf dem Träger hergestellt werden kann. Als halogenfreie Platinmetall-Verbindungen kommen z. B. Platinmetallnitrate, -acetate, -propionate, -butyrate, -oxalate, -carbonate, -oxide, -hydroxide, -acetylacetonate und andere dem Fachmann geläufige in Frage. Als Halogenid enthaltende Verbindungen kommen halogenhaltige Salze und Komplexverbindungen der Elemente der ersten bis fünften Hauptgruppe und der ersten bis achten Nebengruppe des Periodensystems der Elemente (Mendelejew) sowie der Seltenerdmetalle (Atomnummern 58-71) und aliphatische Halogenkohlenwasserstoffe in Frage. Beispiele sind NaBr, NaCl, $MgBr_2$, $MgCl_2$, $AlCl_3$, $CH_2Cl_2$, $NaPF_6$, $MnCl_2$, $MnBr_2$, $CoBr_2$, $CeCl_3$, $SmI_2$, $CuCl_2$, $Na_2ZnCl_4$, $TiCl_4$ und $NR_4Br$, wobei R die oben genannte Bedeutung hat.

Die Menge des Platinmetall-Halogenids oder der das Platinmetall-Halogenid enthaltenden Komplexverbindung im reaktionsbereiten Zustand beträgt 0,01 bis 15 Gew.-%, bevorzugt 0,05 bis 10 Gew.-% gerechnet als Platinmetall und bezogen auf das Gesamtgewicht des Katalysators.

Geeignete Lösungsmittel für die Herstellung von erfindungsgemäßen Träger-Katalysatoren sind z.B. Wasser, aliphatische Kohlenwasserstoffe, wie Pentan, n-Hexan, Cyclohexan usw., aliphatische halogenierte Kohlenwasserstoffe, wie Dichlormethan, Trichlormethan, usw., ungesättigte Kohlenwasserstoffe, wie Penten, Isopren, Cyclopentadien, Hexene, Hexine, Cyclohexene, Cyclooctadiene usw., aromatische Kohlenwasserstoffe, wie Benzol, Toluol, Xylol usw., halogenierte aromatische Kohlenwasserstoffe, wie Chlorbenzol, Dichlorbenzol, usw., primäre, sekundäre oder tertiäre Alkohole, wie Methanol, Ethanol, n-Propanol, iso-Propanol, n-Butanol, iso-Butanol, t-Butanol, Cumylalkohol, iso-Amylalkohol, Diethylenglykol, usw., Ketone, wie Aceton, 2-Butanon, Methylisobutylketon, Acetylaceton usw., Ether, wie Diethylether, Diisopropylether, Methyl-t-butylether, Dioxan, Tetrahydrofuran, usw., Ester, wie Methylacetat, Ethylacetat, usw., Nitrile, wie Acetonitril, Benzonitril, usw., Carbonate, wie Dimethylcarbonat, Diethylcarbonat, Diphenylcarbonat, usw., Dimethylacetamid, N-Methylpyrrolidinon und Tetramethylharnstoff genannt. Selbstverständlich können auch Mischungen solcher Lösungsmittel eingesetzt werden.

Die Herstellung eines erfindungsgemäß verwendeten Katalysators erfolgt nach Methoden, die dem Fachmann grundsätzlich bekannt sind. So können Lösungen eines oder mehrerer der genannten Platinmetalle und der genannten halogenidhaltigen Verbindungen beispielsweise durch Tränken, Adsorption, Tauchen, Sprühen, Imprägnieren und Ionenaustausch auf den erfindungsgemäß einzusetzenden Katalysatorträger gebracht werden.

Es ist weiterhin möglich, ein oder mehrere Platinmetalle und die genannten halogenidhaltigen Verbindungen durch Fällung mit einer Base auf dem Träger zu fixieren. Als Base kommen z.B. (Erd-)-Alkalimetallhydroxide, wie $Ca(OH)_2$, $Mg(OH)_2$, NaOH, LiOH und KOH, (Erd-)-Alkalimetallhydrogencarbonate wie $Ca(HCO_3)_2$, $Mg(HCO_3)_2$, $NaHCO_3$, $LiHCO_3$ und $KHCO_3$, (Erd-)-Alkalimetallcarbonate wie $CaCO_3$, $MgCO_3$, $Na_2CO_3$, $Li_2CO_3$ und $K_2CO_3$, Alkalimetallsalze schwacher organischer Säuren, wie Natriumacetat, Kaliumacetat und Lithiumacetat, und (Erd-)-Alkalimetallsalze von substituierten oder nicht substituierten Phenolen (bei substituierten Phenolen handelt es sich um solche, wie sie weiter unten als in das Verfahren zur Diarylcarbonat-Herstellung einsetzbare beschrieben werden), wie Lithiumphenolat, Natriumphenolat, Natriumkresolat und Kaliumphenolat in Frage. Das Platinmetall und die halogenidhaltige Verbindung können sowohl in beliebiger Reihenfolge nacheinander als auch gleichzeitig auf den Träger gebracht werden. Eine spezielle Ausführungsform der Erfindung beinhaltet das Aufbringen des Platinmetalls durch Fällung eines Platinmetallhalogenids oder einer Platinmetallhalogenid-Komplexverbindung mit einer geeigneten Base (in Frage kommen solche, wie sie oben beschrieben sind), Reduktion der gefällten Platinmetall-Base zum Metall mit einem geeigneten Reduktionsmittel, wie beispielsweise Hydrazin, Formaldehyd, Na-Formiat, NaBH$_4$ bei Temperaturen zwischen 0°C und 200°C oder gasförmigem Wasserstoff bei Temperaturen zwischen 0°C und 500°C, bevorzugt zwischen 20 und 300°C, besonders bevorzugt 30 bis 250°C und Umsetzung des Platinmetalls mit Halogenwasserstoff oder gasförmigem Halogen bei Temperaturen zwischen 20°C und 600°C, bevorzugt 50 und 500°C.

Die Menge Platinmetall in der Lösung zur Herstellung erfindungsgemäß verwendeter Träger-Katalysatoren ist nicht beschränkt, wird bevorzugt aber so bemessen, daß die Konzentration des Metalls in der Lösung 0,001 bis 30 Gew.-%, besonders bevorzugt 0,01 bis 20 Gew.-% beträgt.

Die Temperatur der Lösung vor und während der Umsetzung kann zwischen Schmelzpunkt und Siedepunkt der betreffenden Lösung frei gewählt werden. Bevorzugt wird bei Raumtemperatur gearbeitet. Es kann jedoch zweckmäßig sein, die Lösung vor und/oder während der Umsetzung zu erwärmen, um die Löslichkeit der Platinmetallverbindung zu erhöhen.

Während der Aufbringung der Platinmetallhaltigen Lösung und der Halogenidhaltigen Lösung auf den erfindungsgemäß einzusetzenden Katalysatorträger kann die Mischung gerührt werden. Es kann aber auch vorteilhaft sein, die Mischung stehenzulassen oder zu schütteln, damit gegebenenfalls verwendete Formkörper nicht durch einen Rührer beschädigt werden.

Nach Aufbringung von Platinmetall und Halogenid-haltiger Verbindung auf den erfindungsgemäß einzusetzenden Katalysatorträger wird der Träger-Katalysator z.B. durch Filtrieren, Sedimentieren oder Zentrifugieren abgetrennt. In einer weiteren Ausführungsform der Erfindung wird das Lösungsmittel durch Abdestillieren abgetrennt.

Nach Abtrennung des Lösemittels werden die so erhaltenen Träger-Katalysatoren getrocknet. Dies kann an der Luft, im Vakuum oder im Gasstrom geschehen. Geeignete Gase für die Trocknung des Träger-Katalysators im Gasstrom sind Stickstoff, Sauerstoff, Kohlendioxid und Edelgase sowie beliebige Mischungen der genannten Gase, bevorzugt z.B. Luft. Ebenfalls eignen sich gasförmige Alkene wie Ethen, Propen, Butene, Butadien und Alkine wie Ethin, Propin usw. in beliebiger Zusammensetzung. Die Trocknung erfolgt bei 20 bis 200°C, bevorzugt bei 40 bis 180°C, besonders bevorzugt bei 60 bis 150°C. Die Trockenzeit hängt z.B. von der Porosität des verwendeten Träger und vom verwendeten Lösungsmittel ab. Sie beträgt im allgemeinen einige Stunden, beispielsweise 0,5 bis 50 h, bevorzugt 1 bis 40 h, besonders bevorzugt 1 bis 30 h.

Nach der Trocknung können die getrockneten Träger-Katalysatoren calciniert werden. Dies kann an der Luft, im Vakuum oder im Gasstrom geschehen. Geeignete Gase für die Calcinierung des Träger-Katalysators im Gasstrom sind z.B. Stickstoff, Sauerstoff, Kohlendioxid oder Edelgase sowie beliebige Mischungen der genannten Gase, bevorzugt z.B. Luft. Die Calcinierung erfolgt bei 100 bis 800°C, bevorzugt bei 100 bis 700°C, besonders bevorzugt bei 100 bis 600°C. Während der Calcinierung kann es ggf. von Vorteil sein, wenn man die Zusammensetzung des Gases sprunghaft, z.B. indem man nach 10 Stunden den O$_2$-Gehalt des Calciniergases von 10 auf 20 Vol-% erhöht, oder kontinuierlich ändert, indem man z.B. den Sauerstoffgehalt des Calciniergases innerhalb von 20 Stunden von 0 auf 20 Vol-% mit 1 % Steigerung/h erhöht. Die Calcinierungszeit beträgt im allgemeinen einige Stunden, beispielsweise 0,5 bis 50 h, bevorzugt 1 bis 40 h, besonders bevorzugt 1 bis 30 h.

Bei den erfindungsgemäßen unter Verwendung der genannten Träger-Katalysatoren umsetzbaren aromatischen Hydroxyverbindungen handelt es sich beispielsweise um Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt um Phenol. Allgemein handelt es sich im Falle einer Substitution der aromatischen Hydroxyverbindung um 1 oder 2 Substituenten in der Bedeutung von C$_1$-C$_4$-Alkyl, C$_1$-C$_4$-Alkoxy, Fluor, Chlor oder Brom.

Für die erfindungsgemäße Verwendung können beliebige sowohl organische als auch anorganische Basen oder Mischungen derselben verwendet werden. Als Beispiele für anorganische Basen seien, ohne die erfindungsgemäße Verwendung einzuschränken, Alkalimetallhydroxide und -carbonate, -carboxylate oder andere Salze schwacher Säuren sowie Alkalisalze von aromatischen Hydroxyverbindungen der Formel(II), z. B. Alkalimetallphenolate, genannt. Selbstverständlich können in die erfindungsgemäße Verwendung auch die Hydrate von Alkalimetallphenolaten eingesetzt werden. Als Beispiel für ein solches Hydrat sei hier, ohne die erfindungsgemäße Verwendung einzuschränken, Natriumphenolat-trihydrat genannt. Die Menge an zugesetztem Wasser ist jedoch vorzugsweise so bemessen, daß pro Mol Base maximal 5 Mol Wasser eingesetzt werden. Höhere Wasserkonzentrationen führen i.a. zu schlechteren Umsätzen und Zersetzung gebildeter Carbonate. Als organische Basen seien, ohne die erfindungsgemäße Verwendung einzuschränken, tertiäre Amine, die als organische Reste C$_6$- bis C$_{10}$-Aryl-, C$_7$- bis C$_{12}$-Aralkyl- und/oder C$_1$- bis C$_{20}$-Alkyl-Reste tragen können oder Pyridinbasen oder hydrierte Pyridinbasen darstellen, genannt, beispielsweise Triethylamin, Tripropylamin, Tributylamin, Trioctylamin, Benzyldimethylamin, Dioctylbenzylamin, Dimethylphenethylamin, 1-Dimethylamino-2-phenylpropan, Pyridin, N-Methylpiperidin, 1,2,2,6,6-Pentamethylpiperidin. Bevorzugt wird als Base ein Alkalisalz einer aromatischen Hydroxyverbindung verwendet, besonders bevorzugt ein Alkalisalz der aromatischen Hydroxyverbindung (II), die auch zum organischen Carbonat umgesetzt werden soll. Diese Alkalisalze können Lithium-, Natrium-, Kalium-, Rubidium- oder Cäsiumsalze sein. Bevorzugt werden Lithium-, Natrium-, und Kaliumphenolat, besonders bevorzugt Natriumphenolat eingesetzt.

Die Base kann dem Reaktionsgemisch als reine Verbindung in fester Form oder als Schmelze zugesetzt werden. In einer weiteren Ausführungsform der Erfindung wird die Base dem Reaktionsgemisch als Lösung, die 0,1 bis 80 Gew.-%, bevorzugt 0,5 bis 65 Gew.-%, besonders bevorzugt 1 bis 50 Gew.-% der Base enthält, zugesetzt. Als Lösungsmittel können hierbei sowohl Alkohole oder Phenole, wie z.B. das umzusetzende Phenol (II), als auch inerte Lösungsmittel verwendet werden. Als solche seien die weiter unten als Reaktionmedien erwähnten genannt. Diese Lösungsmittel können allein oder in beliebiger Kombination miteinander eingesetzt werden. So besteht eine Ausführungsform des erfindungsgemäßen Verfahrens beispielsweise darin, daß man die Base in einer Phenolschmelze löst, die mit einem Lösungsmittel verdünnt wurde. Bevorzugt wird die Base in der Schmelze einer aromatischen Hydroxyverbindung gelöst, besonders bevorzugt in einer Schmelze der aromatischen Hydroxyverbindung (II), die zum organischen Carbonat umgesetzt werden soll. Ganz besonders bevorzugt wird die Base, in Phenol gelöst, zugesetzt. Die Base wird in einer von der Stöchiometrie unabhängigen Menge zugesetzt. Das Verhältnis von Platinmetall, z.B. Palladium zu Base wird vorzugsweise so gewählt, daß pro Mol Platinmetall, z. B. Palladium 0,1 bis 500, bevorzugt 0,3 bis 200 besonders bevorzugt 0,9 bis 130 Äquivalente Base, eingesetzt werden.

Die erfindungsgemäße Verwendung wird vorzugsweise ohne Lösungsmittel durchgeführt. Selbstverständlich können auch inerte Lösungsmittel verwendet werden. Als Beispiele für Lösungsmittel seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether genannt.

Bei den im Rahmen der vorliegenden Erfindung eingesetzten quartären Salzen kann es sich beispielsweise um mit organischen Resten substituierte Ammonium- oder Phosphoniumsalze handeln. Geeignet für den Einsatz im erfindungsgemäßen Verfahren sind Ammonium- und Phosphoniumsalze, die als organische Reste $C_6$- bis $C_{10}$-Aryl-, $C_7$- bis $C_{12}$-Aralkyl- und/ oder $C_1$- bis $C_{20}$-Alkyl-Reste und als Anion ein Halogenid, Tetrafluoroborat oder Hexafluorophosphat tragen. Bevorzugt werden in das erfindungsgemäße Verfahren Ammoniumsalze, die als organische Reste $C_6$- bis $C_{10}$-Aryl-, $C_7$- bis $C_{12}$-Aralkyl- und/oder $C_1$- bis $C_{20}$-Alkyl-Reste und als Anion ein Halogenid tragen, eingesetzt, besonders bevorzugt ist Tetrabutylammoniumbromid. Die Menge eines solchen quartären Salzes beträgt 0,1 bis 50 Gew.-%, bezogen auf das Gewicht des Reaktionsgemisches. Vorzugsweise beträgt diese Menge 0,5 bis 15 Gew.-%, besonders bevorzugt 1 bis 5 Gew.-%.

Die erfindungsgemäße Verwendung wird, vorzugsweise ohne Lösungsmittel, bei 30 bis 200°C, bevorzugt bei 30 bis 150°C, besonders bevorzugt bei 40 bis 120°C und bei einem Druck von 1 bis 150 bar, bevorzugt von 2 bis 50 bar, besonders bevorzugt bei 5 bis 25 bar durchgeführt.

Die Träger-Katalysatoren können als Pulver oder Formkörper eingesetzt werden und aus der Reaktionsmischung z.B. durch Filtration, Sedimentation oder Zentrifugieren wieder abgetrennt werden.

Die Herstellung von aromatischen Carbonaten mit den erfindungsgemäß verwendeten Platinmetall-Träger-Katalysatoren kann in verschiedenen Ausführungsvarianten ausgeübt werden. Eine Möglichkeit besteht in der diskontinuierlichen Durchführung. Im Falle einer kontinuierlichen Fahrweise im Gegen- oder Gleichstrom oder in der Rieselphase am Festbett-Katalysator werden Belastungen von 0,01 bis 20 g aromatische Hydroxyverbindung pro Gramm Träger-Katalysator und Stunde, bevorzugt 0,05 bis 10 g aromatische Hydroxyverbindung pro Gramm Träger-Katalysator und Stunde, besonders bevorzugt 0,1 bis 5 g aromatische Hydroxyverbindung pro Gramm Träger-Katalysator und Stunde eingestellt. Die in diskontinuierlichen Versuchen verwendeten Träger-Katalysatoren können bei gleichen Einsatzstoffen ohne Reinigung wiederholt eingesetzt werden. Bei kontinuierlicher Arbeitsweise können die eingesetzten Träger-Katalysatoren über lange Zeit im Reaktor verbleiben. Bevorzugt kommt bei der Verwendung erfindungsgemäßer Träger-Katalysatoren eine kontinuierliche Arbeitsweise im Einzelreaktor oder in einer Kaskade aus Reaktoren zum Einsatz.

Wird der Träger-Katalysator als Pulver eingesetzt, sind zur Vermischung der Reaktionskomponenten die zu verwendenden Rührbehälter mit dafür brauchbaren Rührern ausgestattet. Beim Arbeiten mit Träger-Katalysator-Pulvern als Suspension in Rührgefäßen oder Blasensäulen werden Mengen von 0,001 bis 50 Gew.-%, bevorzugt von 0,01 bis 20 Gew.-%, besonders bevorzugt von 0,1 bis 10 Gew.-% Träger-Katalysator-Pulver, bezogen auf die eingesetzte Menge an aromatischer Hydroxyverbindung, verwendet.

In besonders bevorzugten Ausführungsformen wird der heterogene Träger-Katalysator als Formkörper ortsfest in Rührbehältern, Blasensäulen, einem Rieselphasenreaktor oder Kaskaden dieser Reaktoren eingesetzt. Eine Abtrennung des Träger-Katalysators entfällt dann völlig.

## Beispiel 1:

a) Herstellung eines pulverförmigen Manganoxid-Trägers:

Zu einer Lösung von 126 g Mangan(II)-chlorid (1 mol) in 500 ml Wasser wurden 85 g Natriumhydroxid (2,125 mol), gelöst in 200 ml Wasser, zugetropft. Der so erhaltene Niederschlag wurde abgesaugt, gewaschen und getrocknet. Anschließend wurde er 3h bei 300°C und 2h bei 500°C getempert.

b) Belegung des pulverförmigen Manganoxids mit Palladium:

Zu einer Aufschlämmung von 292,5 g Manganoxid-Pulver in 1500 ml Wasser wurden bei Raumtemperatur 300 ml Lösung von 50 g Natriumtetrachloropalladat(II) mit 15% Palladium in Wasser gegeben. Anschließend wurde mit verdünnter Natronlauge alkalisch gestellt. Die Suspension wurde abgesaugt und bei 100°C getrocknet. Der Kontakt enthielt 2,5 Gew.-% Pd auf Manganoxid-Träger, gerechnet als Metall.

c) Einsatz des Träger-Katalysators zur Herstellung von Diphenylcarbonat:

In einem Autoklaven (1 l) mit Begasungsrührer, Kühler und nachgeschalteter Kühlfalle wurden bei 80°C 8,31 g Tetrabutylammoniumbromid (TBAB) und 0,77 g Mangan(II)acetylacetonat (Mn(acac)$_2$) in 450 g Phenol gelöst. Dann wurden 4 g des oben beschriebenen Träger-Katalysators und 2,21 g Natriumphenolat, gelöst in 50 g Phenol, zugegeben. Unter Einleitung eines Gasgemisch aus Kohlenmonoxid und Sauerstoff (95:5 Vol.-%) wurde dann der Druck auf 10 bar eingestellt. Die Menge an Gasgemisch wurde auf 300 Nl/h eingestellt. Dem Reaktionsgemisch wurde jede Stunde eine Probe entnommen und gaschromatographisch analysiert. Die Analysen ergaben, daß nach einer Stunde 6,6 % Diphenylcarbonat, nach 2 Stunden 12,7 % Diphenylcarbonat und nach 3 Stunden 16,9 % Diphenylcarbonat im Reaktionsgemisch enthalten waren. In der Kühlfalle waren 13,6 g eines Phenol/Wasser-Gemisches kondensiert. Die auf Phenol bezogene Selektivität war größer als 99 %.

**Beispiel 2:**

a) Herstellung von Cer-Manganoxid-Tabletten:

Zu einer Lösung von 997,1 g Cer(III)-chlorid-7-hydrat (2,68 mol) und 1351 g Mangan(II)-chlorid-4-hydrat (6,8 mol) in 17,5 l Wasser wurden bei 85°C 890 g Natriumhydroxid, gelöst in 6 l Wasser, zugegeben. Der Niederschlag wurde abgesaugt, gewaschen, bei 110°C getrocknet und 6h bei 300°C getempert. Der gemahlene Träger wurde mit 4% Graphit vermischt und zu Tabletten verpreßt.

b) Belegung der Cer-Manganoxid-Tabletten mit Palladium:

200 ml Cer-Manganoxid-Tabletten wurden mit 72,5 ml Lösung von 33,3 g Natriumtetrachloropalladat (II) mit 15% Palladium in Wasser getränkt. Anschließend wurde an Luft bei 110°C getrocknet. Der Kontakt enthielt pro Liter 25 g Pd, gerechnet als Metall.

c) Einsatz des Träger-Katalysators zur Herstellung von Diphenylcarbonat:

Der Aktivitätstest erfolgte analog zu Beispiel 1, jedoch mit dem Unterschied, daß der Katalysator ortsfest in einem Maschendrahtkörbchen zum Einsatz kam. Dem Reaktionsgemisch wurde jede Stunde eine Probe entnommen und gaschromatographisch analysiert. Die Analysen ergaben, daß nach einer Stunde 3,7 % Diphenylcarbonat, nach 2 Stunden 8,1 % Diphenylcarbonat und nach 3 Stunden 11,0 % Diphenylcarbonat im Reaktionsgemisch enthalten waren. In der Kühlfalle waren 9,7 g eines Phenol/Wasser-Gemisches kondensiert. Die Selektivität, bezogen auf Phenol, war größer als 99 %.

**Beispiel 3:**

a) Herstellung von Extrudaten aus pulverförmigen Seltenerdoxidgemisch:

Ein kommerziell erhältliches Gemisch aus Seltenerdoxiden (Rhône-Poulenc) wurde mit Wasser angeteigt, extrudiert, 5h bei 110°C getrocknet und 5h bei 400°C calciniert.

b) Belegung der Seltenerdoxid-Extrudate mit Palladium:

200 ml Seltenerdoxid-Extrudat wurden mit 70 ml Lösung von 33,3 g Natriumtetrachloropalladat(II) mit 15% Palladium in Wasser getränkt. Anschließend wurde an Luft bei 110°C getrocknet. Der Kontakt enthielt pro Liter 25 g Pd, gerechnet als Metall.

c) Einsatz des Träger-Katalysators zur Herstellung von Diphenylcarbonat:

Der Einsatz des Träger-Katalysators zur Herstellung von Diphenylcarbonat erfolgte wie in Beispiel 2. Dem Reaktionsgemisch wurde jede Stunde eine Probe entnommen und gaschromatographisch analysiert. Die Analysen ergaben, daß nach einer Stunde 3,4 % Diphenylcarbonat, nach 2 Stunden 7,5 % Diphenylcarbonat und nach 3 Stunden 10,1 % Diphenylcarbonat im Reaktionsgemisch enthalten waren. In der Kühlfalle waren 9,2 g eines Phenol/Wasser-Gemisches kondensiert. Die auf Phenol bezogene Selektivität war unverändert größer als 99 %.

**Beispiel 4:**

a) Belegung der Extrudate aus Beispiel 3 mit Rhodium:

200 ml Seltenerdoxid-Extrudate wurden mit 70 ml Lösung von 12,94 g Rhodium(III)-chloridhydrat in Wasser getränkt. Anschließend wurde an Luft bei 110°C getrocknet. Der Kontakt enthielt pro Liter 25 g Rh, gerechnet als Metall.

b) Einsatz des Träger-Katalysators zur Herstellung von Diphenylcarbonat:

Der Einsatz des Träger-Katalysators zur Herstellung von Diphenylcarbonat erfolgte wie in Beispiel 2. Dem Reaktionsgemisch wurde jede Stunde eine Probe entnommen und gaschromatographisch analysiert. Die Analysen ergaben, daß nach einer Stunde 1,1 % Diphenylcarbonat, nach 2 Stunden 2,4 % Diphenylcarbonat und nach 3 Stunden 3,1 % Diphenylcarbonat im Reaktionsgemisch enthalten waren. In der Kühlfalle waren 3,0 g eines Phenol/Wasser-Gemisches kondensiert.

**Beispiel 5:**

a) Belegung der Extrudate aus Beispiel 3 mit Palladium:

200 ml Seltenerdoxid-Extrudate wurden mit 70 ml Lösung von 12,20 g Bis(acetonitril)-palladium(II)-chlorid in Acetonitril getränkt. Anschließend wurde der Katalysator 5 h bei 1 mbar und 40°C getrocknet. Der Kontakt enthält pro Liter 25 g Pd, gerechnet als Metall.

b) Einsatz des Träger-Katalysators zur Herstellung von Diphenylcarbonat:

In einen Hastelloy C4-Reaktor (h= 100 cm, $d_i$= 1,5 cm) mit ölbeheiztem Heizmantel wurden auf eine Fritte (ca. 10 cm oberhalb des unteren Reaktorendes) 40 ml des oben hergestellten Katalysators gegeben und der Reaktor mit 80 ml inerten Füllkörpern (3x3 mm) aufgefüllt. Der Reaktor war mit einer Gasdosierung, drei Flüssigkeitsdosierungen, einem kontinuierlichem Flüssigkeitsaustrag und einer abgasseitigen Druckhaltung versehen. Das kontinuierlich am oberen Ende des Reaktors ausgeschleuste Abgas durchlief eine dreistufige Kondensationseinheit, bevor es über ein $O_2$-Meßgerät in die Abgasleitung gelangte. Mit Hilfe dreier Pumpen wurden phenolische Lösungen von TBAB, Mn(acac)$_3$ und Naphenolat(NaOPh) kurz oberhalb der inerten Füllkörper so zudosiert, daß pro Stunde 100 g Phenol, 1,7 g TBAB, 0,21 g Mn(acac)$_3$ und 0,44 g NaOPh durch den Reaktor strömten. Unterhalb der Katalysatorschüttung erfolgte die Dosierung von 150 Nl CO/$O_2$-Gemisch/h mit 95 Vol.% CO und 5 Vol.% $O_2$ im Gegenstrom zur Flüssigphase. Die Reaktortemperatur betrug 90°C und der Druck 12 bar.
Dem kontinuierlich ablaufenden flüssigen Reaktionsprodukt wurden stündlich Proben entnommen. Nach 4 Stunden änderte sich die Zusammensetzung des ablaufenden Reaktionsprodukts nicht mehr. Der DPC-Gehalt betrug 11,5 %. Die DPC-Selektivität war auch hier wie in den vorangegangenen Beispielen größer als 99 %. Durch das Reaktionsgas wurden stündlich 16 g eines Phenol/Wasser-Gemisches ausgetragen.

**Beispiel 6:**

a) Belegung von Cerdioxid-Pulver mit Palladium:

Zu einer Lösung von 2,28 g (2,5 mmol) Bis(tetrabutylammonium)tetrabromopalladat in 500 ml analysenreinem Dichlormethan wurden bei Raumtemperatur 25 g Cerdioxid-Pulver (Fa. Strem, Nr.93-5816) gegeben. Dann wurde die Mischung 5 h gerührt und abgesaugt. Der so erhaltene Träger-Katalysator wurde bei 50°C im Vakuum (30 mbar) 17 h getrocknet. Die Bestimmung des Palladiumgehalts durch Atomabsorptionsspektrometrie ergab, daß der Träger-Katalysator 1,0 Gew.-% Palladium (gerechnet als Metall) enthielt.

b) Einsatz des Träger-Katalysators zur Herstellung von Diphenylcarbonat:

Der Einsatz des Träger-Katalysators zur Herstellung von Diphenylcarbonat erfolgte wie in Beispiel 1. Die Analysen ergaben, daß nach einer Stunde 6,1 % Diphenylcarbonat, nach 2 Stunden 11,8 % Diphenylcarbonat und nach 3 Stunden 15,5 % Diphenylcarbonat im Reaktionsgemisch enthalten waren. In der Kühlfalle waren 12,8 g eines Phenol/Wasser-Gemisches kondensiert. Die auf Phenol bezogene Selektivität war größer als 99 %.

**Beispiel 7:**

a) Belegung von Titandioxid-Pulver mit Palladium:

Die Herstellung des Träger-Katalysators erfolgte wie in Beispiel 6, jedoch wurde als Träger 25 g Titandioxid (Fa. Riedel) verwendet. Die Bestimmung des Palladiumgehalts durch Atomabsorptionsspektrometrie ergab, daß der Träger-Katalysator 0,96 Gew.-% Palladium (gerechnet als Metall) enthielt.

b) Einsatz des Träger-Katalysators zur Herstellung von Diphenylcarbonat:

Der Einsatz des Träger-Katalysators zur Herstellung von Diphenylcarbonat erfolgte wie in Beispiel 1, jedoch wurden 6,0 g Träger-Katalysator eingesetzt. Die Analysen ergaben, daß nach einer Stunde 7,3 % Diphenylcarbonat, nach 2 Stunden 12,9 % Diphenylcarbonat und nach 3 Stunden 18,0 % Diphenylcarbonat im Reaktionsgemisch enthalten waren. In der Kühlfalle waren 13,0 g eines Phenol/Wasser-Gemisches kondensiert.

**Beispiel 8:**

a) Belegung von Dysprosiumoxid-Pulver mit Palladium:

Die Herstellung des Träger-Katalysators erfolgte wie in Beispiel 6, jedoch wurde als Träger 25 g Dysprosiumoxid (Fa. Strem, Nr.93-6615) verwendet. Die Bestimmung des Palladiumgehalts durch Atomabsorptionsspektrometrie ergab, daß der Träger-Katalysator 0,92 Gew.-% Palladium (gerechnet als Metall) enthielt.

b) Einsatz des Träger-Katalysators zur Herstellung von Diphenylcarbonat:

Der Einsatz des Träger-Katalysators zur Herstellung von Diphenylcarbonat erfolgte wie in Beispiel 1. Die Analysen ergaben, daß nach einer Stunde 5,4 % Diphenylcarbonat, nach 2 Stunden 10,6 % Diphenylcarbonat und nach 3 Stunden 14,2 % Diphenylcarbonat im Reaktionsgemisch enthalten waren. In der Kühlfalle waren 11,8 g eines Phenol/Wasser-Gemisches kondensiert.

### Beispiel 9

Einsatz des Träger-Katalysators aus Beispiel 7 zur Herstellung von Dikresylcarbonat:

Die Herstellung des aromatischen Carbonats erfolgte wie in Beispiel 7b, jedoch wurde als aromatische Hydroxyverbindung nicht Phenol, sondere para-Kresol eingesetzt. Die Analysen ergaben, daß nach einer Stunde 4,8 % Di-p-kresylcarbonat, nach 2 Stunden 10,8 % Di-p-kresylcarbonat und nach 3 Stunden 14,9 % Di-p-kresylcarbonat im Reaktionsgemisch enthalten waren. In der Kühlfalle waren 10,2 g eines p-Kresol/Wasser-Gemisches kondensiert.

### Beispiel 10

a) Belegung von Titandioxid-Pulver mit Palladium:

Die Herstellung des Träger-Katalysators erfolgte wie in Beispiel 7, jedoch wurde als Träger 25 g Titandioxid (Baytitan, Bayer AG) verwendet. Die Bestimmung des Palladiumgehalts durch Atomabsorptionsspektrometrie ergab, daß der Träger-Katalysator 0,98 Gew.-% Palladium (gerechnet als Metall) enthielt.

b) Einsatz des Träger-Katalysators zur Herstellung von Diphenylcarbonat:

Der Einsatz des Träger-Katalysators zur Herstellung von Diphenylcarbonat erfolgte wie in Beispiel 1, jedoch wurden 5,9 g Träger-Katalysator eingesetzt. Die Analysen ergaben, daß nach einer Stunde 8,6 % Diphenylcarbonat, nach 2 Stunden 14,4 % Diphenylcarbonat und nach 3 Stunden 19,2 % Diphenylcarbonat im Reaktionsgemisch enthalten waren. In der Kühlfalle waren 13,9 g eines Phenol/Wasser-Gemisches kondensiert.

### Vergleichsbeispiel 1:

Einsatz des Träger-Katalysators 5%Pd/C (Fa. Aldrich Nr.20-568-0) zur Herstellung von Diphenylcarbonat:

Der Einsatz des Träger-Katalysators zur Herstellung von Diphenylcarbonat erfolgte wie in Beispiel 1. Die Analysen ergaben, daß nach einer Stunde 1,6 % Diphenylcarbonat, nach 2 Stunden 2,8 % Diphenylcarbonat und nach 3 Stunden 3,1 % Diphenylcarbonat im Reaktionsgemisch enthalten waren. In der Kühlfalle waren 4,0 g eines Phenol/Wasser-Gemisches kondensiert.

### Vergleichsbeispiel 2:

Einsatz des Träger-Katalysators 5%Pd/Al2O3 (Fa. Aldrich Nr.37-148-3) zur Herstellung von Diphenylcarbonat:

Der Einsatz des Träger-Katalysators zur Herstellung von Diphenylcarbonat erfolgte wie in Vergleichsbeispiel 1. Die Analysen ergaben, daß nach einer Stunde 1,4 % Diphenylcarbonat, nach 2 Stunden 2,2 % Diphenylcarbonat und nach 3 Stunden 2,8 % Diphenylcarbonat im Reaktionsgemisch enthalten waren. In der Kühlfalle waren 3,8 g eines Phenol/Wasser-Gemisches kondensiert.

### Patentansprüche

1. Verwendung eines Katalysators enthaltend

(i) ein Platinmetall, eine Platinmetall-Verbindung oder einen, eine Platinmetall-Verbindung enthaltenden, Komplex in einer Menge von 0,01 bis 15 Gew.-% gerechnet als Platinmetall und bezogen auf das Gesamtgewicht des Katalysators;

(ii) Trägermaterial aus einem oder mehreren Oxiden der Metalle TI, V, Mn, Cr, Fe, Co, Ni, Cu, La, Nb, Mo, Pb, der Seitenerdmetalle mit Atomnummern 58 bis71 und der Actiniden der Atomnummern 89 bis 92;

als Katalysator bei der Herstellung eines aromatischen Carbonats der Formel

$$R\text{-}O\text{-}CO\text{-}O\text{-}R,$$

in der

R substituiertes oder nicht substituiertes $C_6$-$C_{12}$-Aryl, bevorzugt substituiertes oder nicht substituiertes Phenyl, besonders bevorzugt nicht substituiertes Phenyl bedeutet,

durch Umsetzung einer aromatischen Hydroxyverbindung der Formel

R-OH,

in der R die obige Bedeutung hat,
mit Kohlenmonoxid und Sauerstoff in Gegenwart eines Cokatalysators, eines quartären Ammonium- oder Phosphonium-Salzes und einer Base bei 30 bis 200°C und bei einem Druck von 1 bis 150 bar.

2. Verwendung gemäß Anspruch 1, **dadurch gekennzeichnet, dass** der Katalysatorträger in Form von ein oder mehreren Metalloxiden aus der Gruppe von V, Mn, Ti, Co, Cu, La, der Seltenerdmetalle (Atomnummern 58 bis 71) sowohl im Sinne chemischer einheitlicher Reinsubstanzen als auch im Gemisch vorliegt.

3. Verwendung nach Anspruch 1, **dadurch gekennzeichnet, dass** als Platinmetall Pd oder Rh, als Metall, Metallhalogenid oder Metallhalogenid enthaltende Komplexverbindung vorliegt.

4. Verwendung nach Anspruch 1, **dadurch gekennzeichnet, dass** man den Trägerkatalysator in einer kontinuierlichen Fahrweise im Gegen- oder Gleichstrom oder mit ortsfesten Katalysatoren in Rührkessel, Blasensäulenreaktoren oder in der Rieselphase am Festbett-Katalysator mit einer Belastung von 0,01 bis 20 g aromatische Hydroxyverbindung pro Gramm Träger-Katalysator und Stunde, bevorzugt 0,05 bis 10 g aromatische Hydroxyverbindung pro Gramm Träger-Katalysator und Stunde, besonders bevorzugt 0,1 bis 5 g aromatische Hydroxyverbindung pro Gramm Träger-Katalysator und Stunde beaufschlagt, und beim Arbeiten als Suspension in Rührgefäßen oder Blasensäulen in Mengen von 0,001 bis 50 Gew.-%, bevorzugt von 0,01 bis 20 Gew.-%, besonders bevorzugt von 0,1 bis 10 Gew.-%, bezogen auf die eingesetzte Menge an aromatischer Hydroxyverbindung, verwendet.

5. Verwendung nach Anspruch 1, **dadurch gekennzeichnet, dass** man als Base ein tertiäres Amin, Alkaliphenolat oder Alkalisalz schwacher Säuren, vorzugsweise Alkalicarboxylate und/oder -phenolate, besonders bevorzugt Natriumphenolat verwendet.

6. Verwendung nach Anspruch 1, **dadurch gekennzeichnet, dass** man als quartäres Salz Tetraalkylammonium- oder -phosphoniumsalze, bevorzugt Tetraalkylammoniumsalze, besonders bevorzugt Tetrabutylammoniumbromid einsetzt.

## Claims

1. The use of a catalyst containing

    (i) a platinum metal, a platinum metal compound or a complex which contains a platinum metal compound in an amount of 0.01 to 15 wt. %, calculated as platinum metal and with respect to the total weight of catalyst;

    (ii) a support material consisting of one or more oxides of the metals Ti, V, Mn, Cr, Fe, Co, Ni, Cu, La, Nb, Mo, Pb, the rare earth metals with atomic numbers 58 to 71 and the actinides with atomic numbers 89 to 92;

    as catalysts when preparing an aromatic carbonate of the formula

    R-O-CO-O-R,

    in which

    R   represents a substituted or unsubstituted $C_6$-$C_{12}$ aryl group, preferably substituted or unsubstituted phenyl, particularly preferably unsubstituted phenyl,

    by reacting an aromatic hydroxy compound of the formula

    R-OH

    in which R is defined in the way given above, with carbon monoxide and oxygen in the presence of a cocatalyst, a quaternary ammonium or phosphonium salt and a base at 30 to 200°C and at a pressure of 1 to 150 bar.

2. The use according to claim 1, **characterised in that** the catalyst support is present in the form of one or more metal oxides from the group consisting of V, Mn, Ti, Co, Cu, La, the rare earth metals (atomic numbers 58 to 71), either in the context of chemically homogeneous pure substances or as a mixture.

3. The use according to claim 1, **characterised in that** Pd or Rh is present as the platinum metal, as a metal, metal halide or a complex compound containing a metal halide.

4. The use according to claim 1, **characterised in that** the supported catalyst is subjected to a load of 0.01 to 20 g of aromatic hydroxy compound per gram of supported catalyst and per hour, preferably 0.05 to 10 g of aromatic hydroxy compound per gram of supported catalyst and per hour, particularly preferably 0.1 to 5 g of aromatic hydroxy compound per gram of supported catalyst and per hour, in a continuous mode of operation in a counter-current or co-current or with positionally-fixed catalysts in stirred tanks, bubble column reactors or in the trickle phase on fixed bed catalysts, and when operating as a suspension in stirred vessels or bubble columns, is used in amounts of 0.001 to 50 wt.%, preferably 0.01 to 20 wt.%, particularly preferably 0.1 to 10 wt.%, with respect to the amount of aromatic hydroxy compound used.

5. The use according to claim 1, **characterised in that** a tertiary amine, alkali metal phenolate or alkali metal salt of a weak acid, preferably an alkali metal carboxylate and/or phenolate, particularly preferably sodium phenolate is used as the base.

6. The use according to claim 1, **characterised in that** a tetraalkylammonium or phosphonium salt, preferably a tetraalkylammonium salt, particularly preferably tetrabutylammonium bromide, is used as a quaternary salt.

**Revendications**

1. Utilisation d'un catalyseur contenant

   (i) un métal du type platine, un composé de métal du type platine ou un complexe contenant un composé de métal du type du platine en une quantité de 0,01 à 15% en poids, calculé comme métal du type du platine et rapporté au poids total du catalyseur ;
   (ii) un support d'un ou plusieurs oxydes des métaux Ti, V, Mn, Cr, Fe, Co, Ni, Cu, La, Nb, Mo, Pb, les métaux de terres rares ayant des nombres atomiques de 58 à 71 et les actinides ayant les nombres atomiques de 89 à 92 ;

   comme catalyseur pour la préparation d'un carbonate aromatique de formule

   R-O-CO-O-R,

   dans laquelle
   R est un groupe aryle en $C_6$-$C_{12}$ substitué ou non substitué, de préférence un groupe phényle substitué ou non substitué, en particulier de préférence un groupe phényle non substitué,
   par réaction d'un composé hydroxy aromatique de formule

   R-O-H,

   dans laquelle R est tel que défini ci-dessus,
   avec du monoxyde de carbone et de l'oxygène en présence d'un co-catalyseur, d'un sel d'ammonium quaternaire ou de phosphonium et d'une base à 30 à 200°C et à une pression de 1 à 150 bar.

2. Utilisation selon la revendication 1, **caractérisée en ce que** le support de catalyseur est sous forme d'un ou plusieurs oxydes de métaux du groupe de V, Mn, Ti, Co, Cu, La, les métaux des terres rares (nombres atomiques de 58 à 71), ainsi que dans le sens de substances pures uniformes chimiquement ou comme mélanges.

3. Utilisation selon la revendication 1, **caractérisée en ce que** le support de catalyseur comprend comme métal du type du platine, Pd ou Rh, sous forme d'un métal, d'un halogénure de métal ou d'un complexe contenant un halogénure de métal.

4. Utilisation selon la revendication 1, **caractérisée en ce qu'**on utilise le catalyseur supporté dans un procédé en continu à contre courant ou selon le même courant ou avec des catalyseurs fixes dans des récipients agités, des réacteurs de colonnes à bulles ou par circulation vers le bas sur un catalyseur dans un lit fixe, avec une charge de 0,01 à 20 g de composé hydroxy aromatique par gramme de catalyseur supporté et par heure, de préférence 0,05 à 10 g de composé hydroxy aromatique par gramme de catalyseur supporté et par heure, en particulier de préférence 0,1 à 5 g de composé hydroxy aromatique par gramme de catalyseur supporté et par heure, et en travaillant avec des suspensions dans des récipients agités ou des colonnes à bulles en des quantités de 0,001 à 50% en poids, de préférence de 0,01 à 20% en poids, en particulier de préférence de 0,1 à 10% en poids, rapporté à la quantité utilisée de composé hydroxy aromatique.

5. Utilisation selon la revendication 1, **caractérisée en ce qu'**on utilise comme base une amine tertiaire, un phénolate alcalin ou un sel alcalin d'acides faibles, de préférence des carboxylates et/ou des phénolates alcalins, en particulier de préférence un phénolate de sodium.

6. Utilisation selon la revendication 1, **caractérisée en ce qu'**on utilise comme sel quaternaire des sels de tétraalkylammonium ou de tétraalkylphosphonium, de préférence des sels de tétraalkylammonium, en particulier de préférence le bromure de tétrabutylammonium.